# EUROPEAN PATENT SPECIFICATION

(11) **EP 3 782 633 B1**
(45) Date of publication and mention of the grant of the patent: **27.03.2024**
(21) Application number: 19868152.0
(22) Date of filing: 04.07.2019
(51) Int. Cl.: A61K 36/22, A61K 8/9789, A61K 47/10, A61K 47/14, A61K 47/26, A61K 47/44, A61P 1/02, A61P 31/04, A61K 9/00, A61K 9/06, A61Q 11/00, A61Q 17/00

(54) **ORAL CAVITY COMPOSITION FOR ANIMALS, AND PERIODONTAL DISEASE-PREVENTING AGENT, INFECTIOUS DISEASE-PREVENTING AGENT, AND HALITOSIS-PREVENTING AGENT FOR ANIMALS**
ORALE ZUSAMMENSETZUNG FÜR TIERE UND MITTEL ZUR VORBEUGUNG VON PARODONTOSE, MITTEL ZUR VORBEUGUNG VON INFEKTIONSKRANKHEITEN UND MITTEL ZUR VORBEUGUNG VON MUNDGERUCH FÜR TIERE
COMPOSITION DE CAVITÉ BUCCALE POUR ANIMAL, ET AGENT DE PRÉVENTION DE MALADIE PARODONTALE, AGENT DE PRÉVENTION DE MALADIE INFECTIEUSE, ET AGENT DE PRÉVENTION DE L'HALITOSE POUR ANIMAL

(43) Date of publication of application: 24.02.2021
(73) Proprietor: SOSIN CO., LTD., Kiyose-Shi Tokyo 204-0022 (JP)
(72) Inventor: WATANABE, Itaru, Kiyose-Shi Tokyo 204-0022 (JP)
(74) Representative: Hoeger, Stellrecht & Partner Patentanwälte mbB
(86) International application number: PCT/JP2019/026615
(87) International publication number: WO 2021/001986

(56) References cited:
- JP-A- 2017 075 098
- JP-A- 2019 077 625
- JP-A- 2019 077 625
- JP-B2- 6 468 559
- AIKATERINI TERMENTZI ET AL: "Natural Resins and Bioactive Natural Products thereof as Potential Anitimicrobial Agents", CURRENT PHARMACEUTICAL DESIGN, vol. 17, no. 13, 1 May 2011 (2011-05-01), pages 1267-1290, XP055404514, NL ISSN: 1381-6128, DOI: 10.2174/138161211795703807
- ISOGAI, E. et al.: "Ceragenine CSA-13 exhibits antimicrobial activity against cariogenic and periodontopathic bacteria", Oral Microbiology and Immunology, vol. 24, no. 2, 2009, pages 170-172, XP055393623, ISSN: 0902-0055, DOI: 10.1111/j.1399-302X.2008.00464.x
- ISOGAI, E. et al.: "Effect of Japanese green tea extract on canine periodontal diseases", Microbial Ecology in Health and Disease, vol. 8, no. 2, 1995, pages 57-61, XP055697187, ISSN: 0891-060X

## Description

### TECHNICAL FIELD

The present invention relates to an oral composition for animal, containing mastic resin and/or mastic essential oil as active components, especially a valid oral composition for pet animals, such as dogs and cats. As well, the present invention also relates to a periodontal disease preventive, an infection disease, and a mouth odor preventive using thereof.

### BACKGROUND ART

In generally case of man (human), an adhesion of dental plaque is as one of causes of dental caries or periodontal disease, and it was indicated that elimination and precaution of dental plaque, i.e., plaque control is important in oral hygiene in the past. A forming mechanism of plaque results that oral microorganism, especially glucosyltransferase which is extracellular enzyme of *Streptococcus mutans,* synthesizes cohesive and insoluble glucans by using glucose as substance whether the glucan adheres to tooth surface and forms plaque which is aggregate of the bacterial cell.

As a method of the plaque control, a mechanically plaque elimination by tooth brash etc. or an intraoral sterilization using oral sterilizer are features. However, in case of the mechanically plaque elimination by tooth brashing etc., it is impossible to substantially remove the plaque without a good and many time tooth brushing on training. As well, in case of using the oral sterilizer, there is a problem that the effect is not enough shown, because the sterilization component does not infiltrate inside towards the aggregate of the bacterial cell. Accordingly, a contrivance, such as increasing the concentration of the sterilization component, lengthening a treatment time, and so on, is necessary. As well, with regard to the plaque elimination by the sterilizer, so the sterilizer are affected with all intraoral fungi and sterilizes oral indigenous bacteria or useful bacteria for human body, therefore, it could not always be satisfied with safety, economy, and efficacy.

As well, the periodontal disease of man is a disease having symptoms causing an inflammation to periodontal tissues, such as gingivitis, periodontitis, and so on. As the pathogen of the periodontal disease, *Porphyromonas gingivalis, Prepovotella intermedia, Treponnema denticola, Candida albicans,* and so on are known. So the periodontal disease is mainly a disease of a plaque derivation, the plaque control is useful for the prevention as with cavity protection, however, an apprehension as with above apprehension is generated. As well, in the periodontal diseases, there are no the disease of the plaque derivation and having further serious condition. They have to be technically treated (mainly such as chemical treatment by antibiotics etc., tooth extraction and so on) in dentist or oral surgery clinic, and there is the apprehension leading to various digestive diseases as adverse reaction according to an administrative agent.

In order to solve the apprehensions of the dental caries and periodontal disease precautions in the above-mentioned man, a development of oral composition with material (for example natural product and so on) which shows an effect with a small amount of active component of which the treatment is easy is variously archived. As one example thereof, an oral composition using mastic (mostly sap thereof) is described in Japanese Patent Publication No.3389556 B (Patent Document 1) and Japanese Patent Publication No.2012-97018 A (Patent Document 2). Mastic shows *Anacardiaceae Pistacia Lentiscus,* and the sap thereof is mainly used in the dental caries and periodontal disease precautions. As well, the mastic sap is known not only the dental caries and periodontal disease precautions in man but also an antibacterial action to *Helicobacter pylori* or *Campylobacter.* As well, the main component of mastic sap is mastic essential oil, masticadienoic acid, isomasticadienoic acid, triterpene, aldehyde, alcohol, poly-β-myrcene, and so on.

Also, as a development of oral composition with material which shows an effect with a small amount of active component of which the treatment is easy, the oral composition mainly using *Lactobacillus brevis* is one of plant Lactobacillus is described in Japanese Patent Publication No.2014-166992 (Patent Document 3). The plant Lactobacillus using in Patent Document 3 is arranged the length of bacterial cell about 0.1~5µm and is used as dead bacterium. Therefore, the oral composition that has a small amount of content and controls a manufacture cost is provided.

By the way, in animals except man, especially pet animals, such as dog, cat, and so on, dental caries and periodontal disease are indispensable. Also, in the pet animals, the chemically treatment, such as a mechanically plaque control using tooth brushing by the owner and a mouth rinsing using an animal periodontal preventive or therapeutic agent, is performed likewise man. As well, a prophylactic treatment, such as the treatment by pet food or chewing gum etc. mixed with their agents, a dose of medicine, or vaccination, is performed. By the way, depending on living condition, pathogenic bacteria causing periodontal and infection diseases of dogs or cats as with man are, for example, *Porphyromonas gingivalis, Prevotella intermedia, Campylobacter rectuas, Tannerella forsynthesis* and so on. However, the non-present bacterium in man, such as *Porphyromonas gulae, Porphyromonas salivosa, Porphyromonas circumdentaria,* and the pathogen of the infection disease living on dog or cat, for example, *Odoribacter denticanis,* or the Pasteurella bacterium, such as *Pasteurella multocida, Pasteurella dagmatis, Pasteurella stomatis* are present.

The prophylactic vaccine for *Porphyromonas gulae* etc. which lives on dog or cat is described in Japanese Patent Publication No.4099213 (Patent Document 4). As well, as the oral composition for *Porphyromonas gulae* etc., the composition derived from an extract of Ashitaba (*Angelica keiskei*) is described in Japanese Patent Publication No.2015-67539 (Patent Document 5). As well, as the oral composition for *Porphyromonas gulae* etc., the composition using mastic is described in Japanese Patent Publication No.6468559 (Patent Document 6).

By the way, as described above, we describe about the Pasteurella bacterium, such as *Pasteurella multocida* and so on, as the pathogen of dog or cat infection diseases except man, however, the infection by the Pasteurella seldom causes the periodontal disease or pneumonia in dog or cat. However, in the case infecting from dog or cat having the Pasteurella to man, there are known that it is highly possible for the man to develop pasteurellosis and thus lead to phlegmon or septicemia. Such a medicament preventing bacterial infections due to *Pasteurella multocida* and so on and a treatment method using the medicament are, for example, described in Japanese Patent Publication No.2018-58875 (Patent Document 7). The invention of Patent Document 7 is described that the effect is shown to effect to the infection disease of animal, such as man, dog, cat, cattle, sheep, goat, pig, chicken, fish horse and so on, whether gram negative or positive does not matter.

JP 2019-077625 A (Patent Document 8) discloses a mouth odor preventing agent for humans and antimicrobial agent for oral cavities for treating and preventing opportunistic infections. The agent contains mastic resin and egg yolk oil as active ingredients.

### THE LIST OF PRIOR ART DOCUMENT

### Patent Document

Patent Document 1: Japanese Patent Publication No.3389556 B
Patent Document 2: Japanese Patent Publication No.2012-97018 A
Patent Document 3: Japanese Patent Publication No.2014-166992 A
Patent Document 4: Japanese Patent Publication No.4099213 B
Patent Document 5: Japanese Patent Publication No.2015-67539 A
Patent Document 6: Japanese Patent Publication No.6468559 B
Patent Document 7: Japanese Patent Publication No.2018-58875 A
Patent Document 8: Japanese Patent Publication No. 2019-077625 A

### SUMMARY OF THE INVENTION

### Problems to be solved by the Invention

Although the inventions of Patent document 1, 2, and 3 show the valid effect for, e.g., *Porphyromonas gingivalis* because being just suitable for man, there are neither description nor suggestion that the inventions show such effect for the pathogen parasitic in dog or cat, such as *Porphyromonas salivosa, Odoribacter denticanis* and so on.

As well, in the invention of Patent document 4, a vaccination is main invention, there is no direct description, thus, the treatment by veterinarian is necessary. On the other words, there are neither description nor suggestion that the pet owner not having the license of veterinarian can take care of dog, cat and so on. Of course, in Patent document 4 there are neither description nor suggestion that mastic is used in Patent documents 1 and 2, and that plant lactobacillus is used in Patent documents 3.

Then, in Patent document 5, a dosage form, such as paste, gel and so on, is adopted, so that the pet owner can easily handle. As well, the description for using mastic is in Patent document 5. In case taking it, the oral composition for animal based on the acknowledge of Patent documents 1-5.

However, the invention of Patent document 5 is that *Angelica keiskei* extract is only active component to *Porphyromonas gulae.* And then, as mentioned above that there is description for using mastic, however, mastic is only used as perfume, and there is neither description nor suggestion that mastic shows the effect to the pathogen of periodontal disease of dog or cat.

As well, as above mentioned, with regard the *Pasteurella,* it is necessary to prevent and sterilize thereof at the stage of parasitic dog or cat, i.e. prevent and sterilize by oral care, because causing the infection disease to other mammals, birds and so on. However, in Patent document 6, there is description that the bactericidal effect is shown to *Porphyromonas gulae,* but is unknown whether the bactericidal effect is shown to *Pasteurella* or not. As well, the invention of Patent document 7 acts in case of just infecting the infection disease (*Pasteurella* disease). Because robenidine (1,2-bis[(E)-(4-cholorophenyl)methylidyneamino]guanidine) is active component and is also prepared by synthesis, it costs in the molecular design or the synthesis of robenidine, and it has a higher risk of side reaction than natural product. And, there are neither description nor suggestion whether it is or not possible for robenidine to use as the oral composition. Furthermore, if the inventions of Patent documents 1-6 are combined with the invention of Patent document 7, there are neither description nor suggestion whether mastic is or not effective for *Pasteurella.*

It is an object of the present invention to provide an oral composition for animal, that the manufacturing cost is controlled by using mastic resin and/or mastic essential oil, in particular the oral composition for animal showing the effect to the periodontal disease bacteria of especially pet animals, such as dog, cat, and so on.

### Means for Solving the Problems

In order to accomplish the present invention, an oral composition for use according to claim 1 is provided.

As well, in order to accomplish the present invention, or wherein said mastic resin is used as a mastic resin solution having 10∼60 weight % of concentrations, or wherein said mastic resin solution is made by solving in solvent, and wherein said solvent for solving said mastic resin is selected from any one of ethanol, glycerin, dipropylene glycol, 1,3-butylene glycol, fatty acid triglyceride (carbon numbers are 8~18 with regard to part of fatty acid derivation), tri (carpylic acid acid/capric acid) glyceryl, fatty acid monoglyceride (carbon numbers are 8~18 with regard to part of fatty acid derivation), mono carpylic acid glyceryl, fatty acid ester (carbon numbers are 8~18 with regard to part of fatty acid derivation), isopropyl myristate, ethyl iso-octanate, octyl dodecyl myristate, higher alcohol (carbon numbers are 8~18), oleyl alcohol, sorbitan fatty acid ester (carbon numbers are 8~18 with regard to part of fatty acid derivation) or sucrose fatty acid ester (carbon numbers are 8~18 with regard to part of fatty acid derivation), or wherein a content of said mastic resin solution is 0.1~50%, or wherein further containing mastic essential oil as an active component, or wherein a content of said mastic essential oil is 0.01-1.0%, or wherein a target animal is dog or cat, or wherein a stay agent is selected from one of the group of liquid paraffin, gelic hydrocarbon that is mixture of liquid paraffin and polyethylene, vegetable oil and yellow bees wax, or wherein stay agents are selected from two or more of the group of liquid paraffin, gelic hydrocarbon that is mixture of liquid paraffin and polyethylene, vegetable oil and yellow bees wax.

In order to accomplish the present invention, a periodontal disease preventive, an infection disease preventive or an odor preventive using the above oral composition for animal.

### ADVANTAGEOUS EFFECTS OF THE INVENTION

According to the oral composition for animal of the present invention, it is clear to control the manufacture cost by using the mastic resin and/or the mastic essential oil and to show the effect to the bacteria (e.g. *Pasteurella multocida* and so on) relative to the periodontal and infection disease of especially pet animals such as dog, cat and so on.

As well, according to the dosage form, it is possible to apply the periodontal preventive or the mouth odor preventive for animal by using the oral composition for animal.

### BRIEF DESCRIPTION OF THE DRAWINGS

In the accompanying drawings:
Figure 1 is a diagram showing a transition of a determination score of a gingivitis index in EXAMPLE (test example 3);
Figure 2 is a graph showing a transition of an intraoral bacterial count; and
Figure 3 is a diagram showing a result of a mouth odor measuring.

### MODE FOR CARRYING OUT THE INVENTION

Hereinafter, an oral composition for animal in the present invention will now be explained in more detail. In the present invention, "mastic sap" indicates a sap gathered from *Pistacia lentiscus* of *the Anacardiaceae,* and its main components are, as mentioned above the section of BACKGROUND ART, masticadienoic acid, isomasticadienoic acid, triterpene, aldehyde, alcohol, poly-β-myrcene, and so on. "Mastic resin" indicates the resin naturally drying and solidifying the mastic sap. "Mastic essential oil" indicates an essential oil made from a volatility component (mainly terpene) with a steam or dry distillation of the mastic sap or the mastic resin. As well, "%" is entirely weight percent in case no special description.

First, the mastic resin will be explained. The mastic resin is an important component showing an antibacterial action to a periodontal bacterium (especially *Porphyromonas gulae*) in the oral composition for animal of the present invention. The mastic resin is used by naturally drying the mastic sap as mentioned above. By the way, the time of the naturally drying is no limited if over 1 day.

Further, in the composition, the mastic resin is used by solving in solvent. The reason for solving the resin in solvent is that the mastic resin itself is not solved in water and is the result of considering a compatibility with various additive agent when the composition takes various dosage forms, such as gel, liquid and so on.

As solvent for solving the mastic resin, it is possible to use ethanol, glycerin, di-propylene glycol, 1,3-butylene glycol, fatty acid tri glyceride (fatty acid part is about 8~18 carbon numbers, preferably 8~12 carbon numbers above all.), tri(caprylic acid/capric acid) glyceryl, fatty acid mono glyceride (fatty acid part is about 8~18 carbon numbers, preferably 8~12 carbon numbers above all.), monocarpic glyceride, fatty acid ester (fatty acid part is about 8~18 carbon numbers, preferably 8~12 carbon numbers above all.), isopropyl myristate, isooctanoic acid ethyl ester, octyl dodecyl myristate, higher alcohol (about 8~22 carbon numbers), oleyl alcohol, sorbitan fatty acid ester (fatty acid part is about 8~18 carbon numbers, preferably 8~12 carbon numbers above all.), sucrose fatty acid ester (fatty acid part is about 8~18 carbon numbers, preferably 8~12 carbon numbers above all.), and so on.

Further, in case using the mastic resin on the oral composition for animal of the present invention, the resin should be a homogeneous solution to the solvent. Hereinafter, the homogeneous solution is called "mastic resin solution". The concentration of the mastic resin solution is preferably 5∼60%. By the way, if the concentration is 5% or below, the bactericidal effect for *Porphyromonas gulae* is not obtained. If the concentration is more than 60%, the mastic resin solution is heterogeneous solution and also decreases the bactericidal effect to *Porphyromonas gulae* not so much as the concentration is below 5%.

The preparation method of the mastic resin solution should be routine method if following above concentration range. Then, the solution temperature of the mastic resin may increase the temperature if considering the boiling point of the solvent and may be in the ordinary temperature in some cases. Furthermore, it is preferable to use as the mastic resin solution after solving the mastic resin to the solvent with filtrating it.

Further, the content of the mastic resin solution is preferably 0.1~50% to the total amount of the oral composition for animal of the present invention. By the way, in the case the content as the mastic resin itself is converted, the bactericidal effect to *Porphyromonas gulae* decreases and does not show if the content of the mastic resin solution is 0.1% or below. As well, if the content of the mastic resin solution is more than 50%, there is concern that a tissue around a diseased site etc. in pet animals cause some kind of an inflammation or allergic reaction, even if the bactericidal effect is enough, and there is also possibility that the mastic resin solution more than 50% decrease the bactericidal effect to *Porphyromonas gulae* lower than the range of 0.1~50% mastic resin solution depending on the situation.

Next, the mastic essential oil will be explained. By the way, as mentioned above, the mastic essential oil should use the oil which refines a volatility component (mainly terpene) by the steam distillation and dry distillation of the mastic sap or resin. As well, refining the volatility component should be routine procedure.

The content of the mastic essential oil is preferably 0.01~1.0% to the total oral composition for animal of the present invention. When the content of the mastic essential oil is more than 1.0%, as with the mastic resin solution, there is concern that a tissue around a diseased site etc. in pet animals cause some kind of the inflammation or allergic reaction, even if the bactericidal effect is enough, and there is also possibility that the mastic resin solution more than 1.0% decrease the bactericidal effect to *Porphyromonas gulae* lower than the range of 0.01~1.0% mastic essential oil depending on the situation. By the way, although the mastic essential oil shows the bactericidal effect to *Porphyromonas gulae,* even if the oil is not contained in the oral composition for animal of the present invention, that is, the mastic resin only contains, the better bactericidal effect is obtained if containing the oil. As well, a role of a preventing mouth odor is carried out with adding the mastic essential oil. As well, if the content of the mastic essential oil is less than 0.01%, the preventing mouth odor is not carried out.

Then, the oral composition for animal of the present invention is archived by containing Lactobacillus that has a mode on particle size distribution 1.0 µm or less, as active component. Here, "mode on particle size distribution" means a value representing an indicator showing a body length of bacterium and a particle length (body length), which is the maximum relative frequency, as for a particle size distribution then measuring the body length of bacterium. In other words, in case "the mode is 1.0 µm or less", it indicates that the body length of bacterium is a range of 0.1-5 µm. By the way, the body length of bacterium can be measured by general application, e.g. electron microscope and so on. Although the lactobacillus shows a deactive effect to *Porphyromonas gulae,* in case that the mode is more than 1.0 µm, the lactobacillus is used by 1.0 µm or less because a fetching number to the bacteria drastically decreases. As well, the Lactobacillus using the present invention should be prepared by using a known art (e.g. see WO2009/157073).

The lactobacillus using for the oral composition for animal of the present invention is lactobacillus such as *Lactobacillus brevis(L. brevis), Lactobacillus brevis subspecies coagulans (L. brevis subspecies coagulans), Lactobacillus acidphilus (L. acidphilus), Lactobacillus gasseri (L. gasseri), Lactobacillus mali (L. mali), Lactobacillus plantarum (L. plantarum),Lactobacillus buchneri(L. buchneri), Lactobacillus casei (L. casei), Lactobacillus johnsonii (L. johnsonii), Lactobacillus gallinarum (L. gallinarum), Lactobacillus amylovorus (L. amylovorus), Lactobacillus rhamnosus (L. rhamnosus), Lactobacillus kefir(L. kefir), Lactobacillus paracasei(L. paracasei), Lactobacillus crispatus(L. crispatus), Lactobacillus lactis (L. lactis),* and so on, Enterococcus bacteria such as *Enterococcus faecalis, Enterococcus faecium* and so on, and Bifidobacterium bacteria such as *Bifidobacterium bifidum, Bifidobacterium longum (B.longum), Bifidobacterium adolescentis (B. adolescentis), Bifidobacterium infantis (B. infantis), Bifidobacterium breve (B. breve)*, *Bifidobacterium catenulatum (B. catenulatum),* and so on. The Lactobacillus bacteria are preferable among them, and the strain of *Lactobacillus brevis FERM BP-4693* is further preferably as bacterial strain among them. As well, it is preferable for the lactobacillus concerned to use bacillus mort. For the reason, it is easy for the bacillus mort to prepare when using as the lactobacillus of the present invention, and the bacillus mort also substantially shows the preferable bactericidal effect.

As well, as for said lactobacillus, it is preferable to incorporate 0.01-1.0% for the gross quantity of the oral compositions for the said oral composition for animals for the animal concerned with this invention. If the lactobacillus is less than 0.01%, the effect for sterilizing *Porphyromonas gulae* is not shown. As well, if the lactobacillus is more than 1.0%, it will have an effect in the fetching number of said bacteria.

As well, a dosage form of the oral composition for animal of the present invention may be selected from toothpaste agent, gel, liquid tooth agent, powder tooth agent, mouthwash, film agent, chewing gum, pet food additive, or dermatilogic paste.

As well, it is possible to further prepare an animal periodontal preventive and an animal mouth odor preventive on similar blending condition of the oral composition for animal of the present invention.

As mentioned above embodiment, although it is possible to carry out the oral composition for animal of the present invention and the animal periodontal preventive and mouth odor preventive using the composition, various additives should be contained. The additives will be explained as follows.

As one example of the additives, the oral composition for animal is made by further blending papaya extract and/or chitosan. They shows multiplier effect with the above lactobacillus.

The papaya extract is an extract derived from a natural papaya fruit and the extract which is extracted by grinding the natural papaya fruit and soaking it in solvent such as ethanol and so on. The papaya fruit should be ripe one or unripe one which is still green. Whether the papaya extract serves as moistening agent and can keep moisture in oral, the immature papaya plentifully contains papaya enzyme especially. The papaya enzyme facilitates to remove tooth plaque on tooth surface or between tooth and gum and further shows the effect that the lactobacillus deactivates *Porphyromonas gulae.* Although the content of the papaya extract is particularly no restriction, the content is preferably 0.005%~10% to the total amount of the oral composition of the present invention. The above effect is not shown if less than 0.005%, and it has the possibility that an effect of the lactobacillus to *Porphyromonas gulae* become weak if excessively more than 10%.

In contrast, chitosan is obtained by boiling and processing chitin obtained from an external skeleton of crustaceans, such as crab, shrimp and so on, with using strong base. Because chitosan is polysaccharide, it is not only used as adhesive but also shows an antibiotic or a coating effect of tooth surface. As well, it is possible to keep the above lactobacillus or the papaya enzyme on the tooth surface at long time. Herewith, the deactivation effect of the papaya enzyme is further shown. Although the content of the chitosan is particularly no limited, the content is preferably 0.005~10% to the total oral composition of the present invention. The above effect is not shown if less than 0.005%, and it has the possibility that an effect of the lactobacillus to *Porphyromonas gulae* become weak if excessively more than 10%.

Furthermore, chitosan and papaya extract also should be formulated at the same time. Herewith, chitosan can lengthen the time for providing the papaya enzyme and lactobacillus to the tooth surface or between tooth and gum, and the deactivation effect of the papaya enzyme to *Porphyromonas gulae* is further shown by formulating chitosan. Although the content of chitosan and the papaya extract is particularly no limited, the content is preferably 0.005~10% to the whole oral composition of the present invention. The above effect is not shown if less than 0.005%, and it has the possibility that an effect of the lactobacillus to *Porphyromonas gulae* become weak if excessively more than 10%.

The abrasive(s) is/are selected from silica-base abrasive such as silica gel, precipitated silica, additivity silica, hydrous silicic acid, anhydrous silicic acid, zeolite, aluminosilicate, zirconosilicate and so on, crystal cellulose, dibasic calcium phosphate dihydrate, dibasic calcium phosphate anhydrate, calcium pyrophospahte, tribasic magnesium phosphate, tribasic calcium phosphate, aluminium hydroxylate, alumina, light calcium carbonate, heavy calcium carbonate, magnesium carbonate, zirconium silicate, synthetic resin abrasive and so on. It is possible to simultaneously use one kind of them or more than two kinds of them. The amounts of these abrasives are generally 0~60% to the oral composition of the present invention.

The humectant(s) is/are selected from polyvalence alcohol such as glycerin, conc.glycerin, diglycerin, sorbit multitol, dipropylene glycol, propylene glycol, 1,3-butylene glycol, xylitol, polyethylene glycol and so on, and one kind of them or two kinds of them can be used. As well, these can use as the solvent solving the mastic resin.

The binder(s) (thickener(s)) is/are selected from carrageenan, alginic acid and the derivative such as sodium alginate, alginic acid propylene glycol ester, sodium alginate containing calcium, potassium alginate, calcium alginate, ammonium alginate and so on, xanthan gum, gum guaiac, gelatin, agar, sodium carboxymethylcellulose, hydroxyethylcellulose, hydroxypropylcellulose, sodium poly acrylate, pullulan, and so on, and it is possible to use one kind of them or more than two kinds of them. As well, the thickener also has the role as gel agent.

The foaming agent(s) is/are selected from sodium lauryl sulfate, sodium lauroylsarcosine, sodium alkyl sulfosuccinate, sodium palm oil sodium sulfonate glycerin monoester, sodium α-olefin sulfonate, N-acyl amino acid salt such as N-acyl glutamate, and so on, 2-alkyl-N-carboxymethyl-N-hydroxyethyl imidazolium betaine, maltitol fatty acid ester, sucrose fatty acid ester, poly glycerin fatty acid ester, fatty acid ethanol amide, polyoxyethylene sorbitan monostearate, polyoxyethylene hardened castor oil, polyoxyethylene fatty acid ester, glycerin fatty acid ester, and so on, and it is possible to use one kind of them or more than two kinds of them.

The pH (hydrogen ion concentration) modifier is selected from citric acid, (mono- or di-) sodium citrate, malic acid, (mono- or di-) sodium malate, gluconic acid, (mono- or di-) sodium gluconate, succinic acid, sodium succinate, lactic acid, (mono- or di-) sodium lactate, potassium carbonate, sodium hydrogen carbonate, and so on, it is possible to use one kind of them or more than two kinds of them.

As the retention agent for retaining the active component in the oral composition for animal of the present invention, it is possible to use selected from liquid paraffin, gelatinized hydrocarbon which is a mixture of liquid paraffin and polyethylene, vegetable oil, yellow bees wax, and so on and combined use one kind of them or more than two kinds of them. As well, said gelatinized hydrocarbon also plays a role as gelatinizer.

The sweetener is sodium saccharin, aspartame, trehalose, stevioside, stevia extract, p-methoxy cynnamaldehyde, neohesperidin dihydrochalcone, periraltin, xylitol and so on.

The preserve is parabens such as methyl paraben, ethyl paraben, propyl paraben, butyl paraben and so on, sodium benzoate, phenoxy ethanol, alkyldiaminoethylglycine hydrochloride, and so on.

As the aroma chemical component, it is possible to combined use one kind or more than two kinds of the components selected from l-menthol, anethole, menthon, cineol, limonene, carvone, methyl salicylate, ethyl butyrate, eugenol, thymol, cinnamaldehyde, trans-2-hexenal, and so on. These components should combine separately, however, essential oil containing said components should be used.

By the way, although the content of the respective components as mentioned above humectant, binder, foaming agent, retention agent, sweetener, preserve, aroma chemical component, and so on, is no limited , the content is generally 0.001~20% to the whole oral composition for animal.

As well, fatty alcohol or ester thereof, terpenoid hydrocarbon or terpenoid alcohol, phenol ether, aldehyde, ketone, an aroma component such as lactone and so on, essential oil should be mixed at the range not obstructing the effect of the present invention in addition to said aroma chemical component. The content of the aroma component is generally 0.001~20% to the whole oral composition for animal.

In the oral composition for animal of the present invention, other further effective component should be mixed. Such component(s) is/are selected from lysozyme chloride, sodium fluoride, potassium fluoride, sodium monofluorophosphate, potassium nitrate, sodium poly phosphate, poly ethylene glycol, poly vinyl-N-pyrrolidone, hinokithiol, ascorbic acid (vitamin C), ascorbic acid salt derivative, chlorhexidine salt, cetylpyridinium chloride, benzalkonium chloride, benzetonium chloride, bisabolol, triclosan, isopropylmetylphenol, tocopherol, tocopherol acetate, ε-amino caproic acid, tranexamic acid, aluminium hydroxyl allantoin, aluminium lactate, dihydrocholesterol, glycyrrhetinic acid, glycyrrhizic acid salt derivative, chlorophyll copper complex salt, sodium chloride, guaiazulenesulfonate salt, dextranase, pyridoxine hydrochloride, medicated hydroxyl apatite, and so on, and it is possible to mix one kind of them or more than two kinds of them. The content of the effective component is generally 0.001~20% to the whole oral composition for animal.

Then, in case mixing the above mastic resin (however as mastic resin solution), the mastic essential oil, lactobacillus, additive etc. on the above range toward the whole oral composition for animal of the present invention, the residue should be used as solvent (e.g. the solvent for solving the mastic resin), gelatinizer, and so on. Also, with regard to the additives, it is possible to be applied to the periodontal disease preventive or mouth odor preventive for animal by the mixture similar to the oral composition for animal of the present invention.

The oral composition for animal of the present invention can prepare in proportion to the general method, and the method is no particularly limited.

As well, although the oral composition for animal of the present invention described various embodiments in case being suitable for *Porphyromonas gulae,* we will think the composition bites into the periodontal disease bacteria ever presenting in mammals except human, e.g. *Porphyromonas salivosa, Odoribacter denticanis* and *Pasteurella* fungi and so on.

As well, although the oral composition for animal of the present invention has explained the embodiments as object of the animals except human, i.e. dog or cat, it is also possible to use rabbit, hamster, guinea pig, and so on as pet.

As presented above, the oral composition for animal in the present invention are explained, the embodiments of the present invention are no limited.

### EXAMPLES

Examples are further explained to confirm above embodiments of the oral composition for animal in the present invention. Here, with regard to the examples, we examine the effect between the periodontal disease bacterium (*Porphyromonas gulae* in the example. Hereinafter, so-called "P. *gulae*".) and the components of the oral composition for animal of the present invention such as the mastic resin, the mastic essential oil and lactobacillus of which the frequency value in the particle size distribution is 1.0 mm or less (hereinafter, "nano-type lactobacillus" in the present example).

### [Preparation Example 1] Preparations of the mastic resin and the mastic essential oil

First, sap collecting from *Pistacia lentiscus* in Chios of Greece (hereinafter, so-called "mastic sap") was naturally dried on one day. After drying, a volatility component and a resinoid material was separated from the mastic sap with steam distillation, and thus the resinoid material of them was "the mastic resin" using on the example of the present invention. On the other hand, said volatility component was "the mastic essential oil" using on the example of the present invention example by further refining after isolating by steam distillation. Further, it is subjected to the general method for refining oil.

Next, the mastic resin was a solution having 30% concentration with using tri (caprylic acid/capric acid) as solvent. The obtained solution was "mastic resin solution" which is on the present examples.

### [Preparation Example 2] Preparations of nano-type lactobacillus

The nano-type lactobacillus using in the examples was prepared in accordance with WO2009/157073.

First, *Lactobacillus brevis* (strain is FERM BP-4693. Hereinafter, simply so-called "bacillus brevis") which is plant lactobacillus was used as said lactobacillus, and said bacillus brevis was cultivated on known nutrient medium adding 5% glucose at 36.5 °C by using 20% sodium hydroxide aqueous solution while controlling pH (hydrogen ion concentration) at cultivation to pH 6.5. Then, the cultivation was finished when the consumption of glucose had completed.

After the cultivation, the cultivation liquid is heat treated at 80°C on 10 minutes, wherein the bacterial cell was washed by PBS (phosphate buffer solution), wherein four times amount of dextrin to the bacterial cell was added as diluting agent, wherein the sample was prepared by freeze-dried after dispersing by mixer, and wherein the sample dispersed in PBS so as to 10 mg/mL of bacterial cell concentration again. As well, the nano-type lactobacillus (bacillus brevis) using in the Example of the present invention was the pH holding on 6.5 when manufacturing process. Furthermore, the nano-type lactobacillus was killed bacteria by heat-treating.

By the way, when the particle size of the prepared nano-type lactobacillus was determined, all bacterial cells were 0.7 to 1.0 µm or less, and the mode of particle size distribution was 1.0 µm or less. As well, the determination of the particle size distribution was depending on the general method.

### [Preparation Example 3] Preparations of the respective dilutents of mastic resin solution, mastic essential oil and nano-type lactobacillus

Furthermore, mastic resin solution, mastic essential oil and nano-type lactobacillus using in the EXAMPLE were respectively diluted by using tri (caprylic acid/capric acid) as solvent. In particular, the mastic resin solution diluted tenfold, and the mastic essential oil and the nano-type lactobacillus respectively diluted hundredfold. Hereinafter, the respective components are called "mastic resin solution diluent", "mastic essential oil diluent" and "nano-type lactobacillus diluent". Further, the test tube etc. using on dilution used the sterilized thereof. As well, the respective diluents are prepared at time of use.

### [Preparation Example 4] Preparation of test solution for the effect test against anti-periodontal disease bacteria

As the representative example of the test solution for the effect test against anti-periodontal disease bacteria of the present EXAMPLE (simply, there is so-called "test solution"), for example, a test solution of EXMPLE 8 later will be explained. Although EXAMPLE 8 shows "mastic resin solution diluent 3.00%, mastic essential oil 0.03%, nano-type lactobacillus 0.01%" (see Table 2), in this case should be the total amount of 9 mL by adding the mastic resin solution diluent 2.7 mL, the mastic essential oil 2.7 mL and the nano-type lactobacillus diluent 1.0 mL which are respectively prepared at Preparation example 3 and tri (caprylic acid/capric acid) glyceryl 2.6 mL. By the way, the mixing order is, in particular, no limited.

By the way, if the later respective examples (see Table 1 to 4) keep that the total amount is 9 mL, the amount of the respective diluents and tri (caprylic acid/capric acid) glyceryl can be suitably changed by matching the respective concentrations of the mastic resin solution, the mastic essential oil and the nano-type lactobacillus.

As well, a test solution of control test (comparative example. See Table 1 to 4.) does not mix the mastic resin solution, the mastic essential oil and the nano-type lactobacillus, but only is tri (caprylic acid/capric acid) glyceryl. As well, the detail will be explained later.

As well, the test solutions of the respective examples and the comparative example were also prepared at time of use.

### [Preparation Example 5] Preparation of the periodontal disease bacterium (P. gulae) solution

As the periodontal disease bacterium, *P. gulae* using in test example 1 later was selected, and a bacterial strain was JCM 3865^{T}.

Then, the bacterial strain was anaerobic-cultivated on blood agar medium at 37°C 5 days. After the cultivating, the growth colony was collected by platinum loop and was suspended in sterilized physiological saline and McFarland turbidity standard solution (NO.1). The suspension solution is "periodontal disease bacterium (*P*. *gulae)* solution" using in the example. As well, the number of *P*. *gulae* solution was 1~3X10⁸CFU/mL. By the way, the periodontal disease *P*. *gulae* solution was prepared at time of use.

### [Test Example 1] anti periodontal disease bacterium effect test Part 1

The test was done by using the test solutions for the periodontal disease bacterium effect test of the respective example and the test example prepared in Preparation example 3 and the *P. gulae* solution prepared in Preparation example 5.

First, the test solution of the respective practical example will be explained. "practical examples 1 to 4" respectively varified the variations of the mastic essential oil (the concentration fixes 0.03%.) and the nano-type lactobacillus (the concentration fixes 0.01%.) with fixing the mastic resin solution to 10.0%. "practical examples 5 to 8" respectively varified the variations of the mastic essential oil (the concentration fixes 0.03%.) and the nano-type lactobacillus (the concentration fixes 0.01%.) with fixing the mastic resin solution to 3.0%. "practical examples 9 to 12" respectively varified the variations of the mastic essential oil (the concentration fixes 0.03%.) and the nano-type lactobacillus (the concentration fixes 0.01%.) with fixing the mastic resin solution to 1.0%. "practical examples 13 to 16" respectively varified the variations of the mastic essential oil (the concentration fixes 0.03%.) and the nano-type lactobacillus (the concentration fixes 0.01%.) with fixing the mastic resin solution to 0.5%. Further, the preparation of the test solution of the respective examples was prepared at time of use by the method showing in said Preparation example 3.

Next, the respective comparative examples will be explained. "comparative example 1" is as the control experiment of the practical examples 1 and 2, "comparative example 2" is as the control experiment of the practical examples 3 and 4, "comparative example 3" is as the control experiment of the practical examples 5 and 6, "comparative example 4" is as the control experiment of the practical example 7, "comparative example 5" is as the control experiment of the practical example 8, "comparative example 6" is as the control experiment of the practical example 9, "comparative example 7" is as the control experiment of the practical example 10, "comparative example 8" is as the control experiment of the practical examples 11 and 12, "comparative example 9" is as the control experiment of the practical examples 13 and 14, "comparative example 10" is as the control experiment of the practical examples 15 and 16, "comparative example 1" is as the control experiment of the practical example 1 and 2, the respective experiment was performed.

Next, the anti-periodontal disease bacterium effect test will be explained. Directly, the test is to chronologically measure the number of *P. gulae* at the point of time when adding 1 mL of *P. gulae* solution to 9 mL of the respective test solution of the respective practical example as the reaction time 0 minute. Next, the test will be concretely explained.

First, when respectively adding 1 mL of *P. gulae* solution to 9 mL of the respective test solution of the respective practical examples 1 to 16 (Reaction time 0 minute), 1mL of its mixed solution was collected by pipet. The mixed solution was added with 9 mL of the sterilized saline and thereby gradually diluting at ten times. The diluted mixed solution, which is thought to be suitable dilution step, was collected 0.1mL each, and was smeared on two blood agar mediums each. After the smearing, the respective medium was anaerobic cultivated at 37 °C, 5 days, thereafter, the colony count of the respective medium was measured and thereby calculating the number of fungi of *P. gulae* at the reaction time 0 minute in the respective practical examples.

Under similar conditions, the respective number of *P. gulae* was measured in 0, 5, 10, 15 and 30 minutes after reacting the test solutions of the practical examples 1 to 16 and the *P. gulae* solution. Provided, there were no measuring with regard to the reaction time 5 minutes of the practical example 7, the reaction time 5 minutes of the practical example 9, and the reaction time 30 minutes of the practical example 11 for convenience (see as stated below Table 2 and 3).

After the measuring of the number, with regard to the practical examples 5 to 8, under similar conditions, the respective number of *P. gulae* was measured in 0, 5, 10, 15 and 30 minutes after reacting the test solutions and the *P. gulae* solution again(at twice). Provided, after the measuring of the number, with regard to the practical examples 1 to 4, under similar conditions, the respective number of *P. gulae* was measured in 0, 1, 3 and 5 minutes after reacting the test solutions and the *P. gulae* solution at twice. As well, with regard to the practical examples 9 to 16, the respective number of *P. gulae* was not measured at twice (see as stated below Table 3 and 4).

Next, with regard to the comparative examples 1 to 10, the test is to chronologically measure the number of *P*. *gulae* at the point of time when adding 1 mL of *P. gulae* solution to 9 mL of only tri (caprylic acid/capric acid) glyceryl without the mastic resin solution, the mastic essential oil and the nano-type lactobacillus as the reaction time 0 minute at the similar conditions, such as dilution condition, medium condition and reaction time of the respective practical examples.

Then, the variations of the practical examples 1 to 16 and the comparative examples 1 to 10 will be shown in Tables 1 to 4.

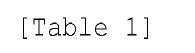

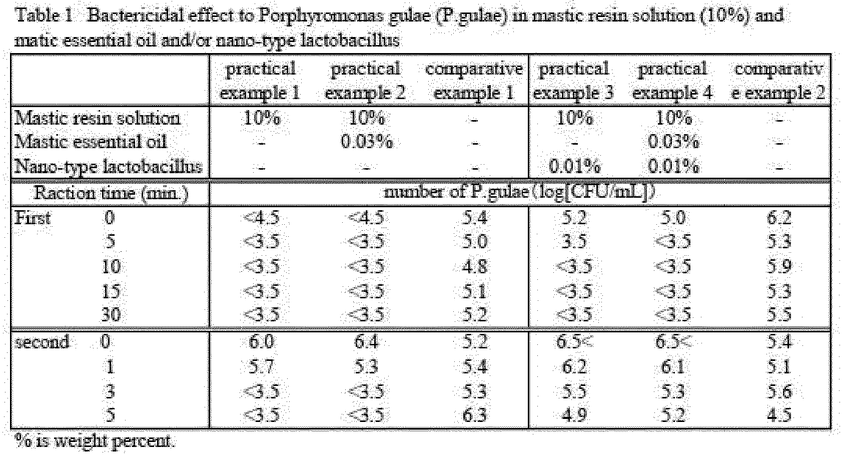

First, Table 1 will be explained. Table 1 is a measuring result of the number of *Porphyromonas gulae (P. gulae)* when respectively changing between the mastic essential oil and the nano-type lactobacillus with fixing the practical examples 1 to 4, i.e. the mastic resin solution to 10%. As well, the bacterium number translates into common logarithm, so that the figure length of the bacterium number becomes clear.

In the comparative example 1, the bacterium number is gradually one digit decreasing or approximately constant, that is the variation of the number is not much, however, in the practical example 1 only containing 10% of the mastic resin solution and the practical example 2 containing 10% of the mastic resin solution and 0.03% of the mastic essential oil, an utilizing instantaneous figure length reduced about 4 (before the reaction between the test solution and *P. gulae* solution is about log [CFU/mL]=8) and was below the measuring range threshold (log [CFU/mL]<3.5) 5 minutes later. Then, in the practical examples 1 and 2, when the bacterium number was measured again, it understood that the bacterium number was below the measuring range threshold (log [CFU/mL]<3.5) at reaction time 3 minutes (see Table 1 "2^{nd}").

Next, in the comparative example 2, the bacterium number is also gradually one digit decreasing or approximately constant, that is the variation of the number is not much, however, in the practical example 3 containing 10% of the mastic resin solution and 0.01% of the nano-type lactobacillus and the practical example 4 containing 10% of the mastic resin solution, 0.03% of the mastic essential oil and 0.01% of the nano-type lactobacillus, *P. gulae* solution is about log [CFU/mL]=8) and was below the measuring range threshold (log [CFU/mL]<3.5) at the reaction time 5~10 minutes on first measuring. Then, in the practical examples 3 and 4, when the bacterium number was measured again, it understood that the bacterium number was slowly reducing at reaction time 5 minutes different from the practical example 1 and 2 (see Table 1 "2^{nd} time").

From this thing, when mixing practical examples 1-4 namely the mastic resin solution with the mastic essential oil and/or nano-type lactobacillus alone itself when I set to 10 percent, there was the number of the bacteria of *P*. *gulae* in a tendency to decrease, and it was revealed that a measurement range limit (log [CFU/mL] <3.5) was as follows surely 30 minutes later.

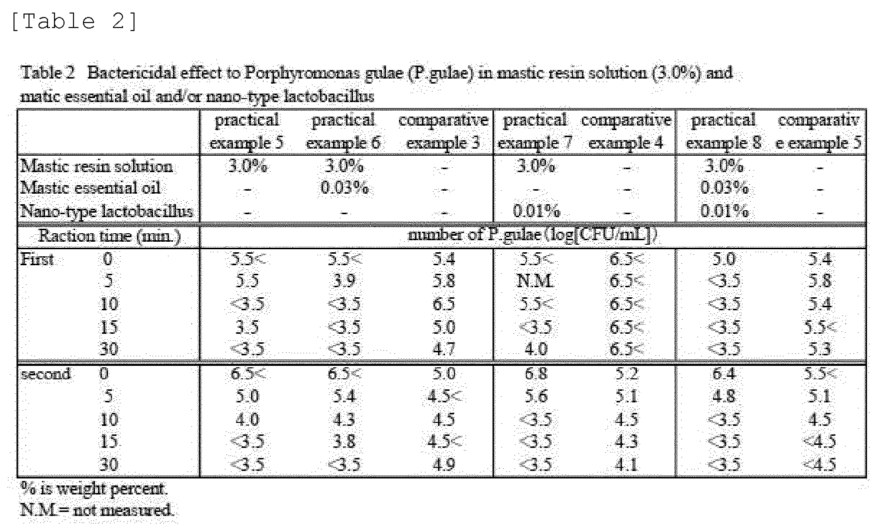

Next, Table 2 will be explained. Table 2 is a measuring result of the number of *Porphyromonas gulae (P. gulae)* when respectively changing between the mastic essential oil and the nano-type lactobacillus with fixing the practical examples 5 to 8, i.e. the mastic resin solution to 3.0%. As well, the bacterium number translates into common logarithm similar to Table 1, so that the figure length of the bacterium number becomes clear.

In the comparative example 3, the bacterium number is gradually one digit decreasing or approximately constant, that is the variation of the number is not much, however, in the practical example 5 only containing 3.0% of the mastic resin solution and the practical example 6 containing 3.0% of the mastic resin solution and 0.03% of the mastic essential oil, an utilizing instantaneous figure length reduced about 2.5 (before the reaction between the test solution and *P. gulae* solution is about log [CFU/mL]=8) and was below the measuring range threshold (log [CFU/mL]<3.5) 10 minutes later. Then, in the practical examples 5 and 6, when the bacterium number was measured again, it understood that the bacterium number was below the measuring range threshold at reaction time 15 minutes and both below the measuring range threshold at reaction time 30 minutes (see Table 2 "2^{nd} time").

Next, in the comparative examples 3 and 4, the bacterium number is also gradually one digit decreasing or approximately constant, that is the variation of the number is not much, however, in the practical example 7 containing 3.0% of the mastic resin solution and 0.01% of the nano-type lactobacillus and the practical example 8 containing 3.0% of the mastic resin solution, 0.03% of the mastic essential oil and 0.01% of the nano-type lactobacillus, *P*. *gulae* solution is about log [CFU/mL]=8) and was below the measuring range threshold (log [CFU/mL]<3.5) at the reaction time 30 minutes on first measuring. However, in the practical examples 7 and 8, when the bacterium number was measured again, it understood that the bacterium number was slowly reducing at reaction time 10 minutes different from the practical example 5 and 6(see Table 2 "2^{nd} time").

From this thing, when mixing practical examples 5-8 namely the mastic resin solution with the mastic essential oil and/or nano-type lactobacillus alone itself when I set to 3.0%, there was the number of the bacteria of *P. gulae* in a tendency to decrease, and it was revealed that a measurement range limit (log [CFU/mL] <3.5) was as follows surely 10 minutes later.

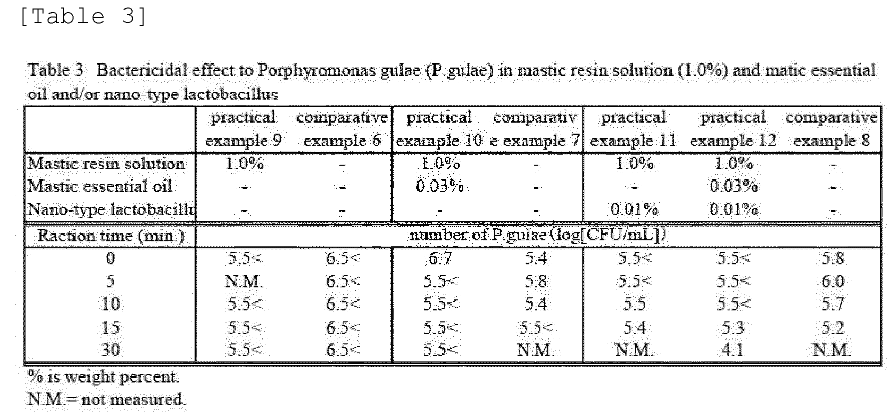

Next, Table 3 will be explained. Table 3 is a measuring result of the number of *Porphyromonas gulae (P. gulae)* when respectively changing between the mastic essential oil and the nano-type lactobacillus with fixing the practical examples 9 to 12, i.e. the mastic resin solution to 1.0%. As well, the bacterium number translates into common logarithm similar to Tables 1 and 2, so that the figure length of the bacterium number becomes clear.

In the comparative examples 6 and 7, the bacterium number is gradually one digit decreasing or approximately constant, that is the variation of the number is not much, however, in the practical example 9 only containing 1.0% of the mastic resin solution and the practical example 10 containing 1.0% of the mastic resin solution and 0.03% of the mastic essential oil, the bacterium number is gradually one digit decreasing or log [CFU/mL]>5.5 even if decreasing.

Next, In the comparative example 8, the bacterium number is gradually one digit decreasing or approximately constant, that is the variation of the number is not much, however, in the practical example 11 containing 1.0% of the mastic resin solution and 0.01% of the nano-type lactobacillus and the practical example 12 containing 1.0% of the mastic resin solution, 0.03% of the mastic essential oil and and 0.01% of the nano-type lactobacillus, the bacterium number is slightly more reduced than the practical examples 9 and 10.

From this thing, when, practical examples 9-12, i.e. the mastic resin solution was set to 1.0%, there was the number of the bacteria of *P. gulae* in a tendency to decrease. However, it understood that the practical examples 9-12 are slightly reducing of the bacterium number or slightly constant different from the practical examples 1 to 8.

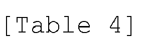

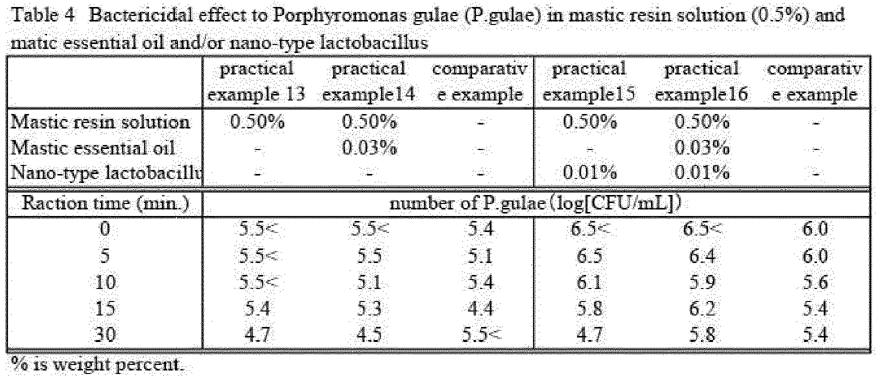

Next, Table 4 will be explained. Table 4 is a measuring result of the number of *Porphyromonas gulae (P. gulae)* when respectively changing between the mastic essential oil and the nano-type lactobacillus with fixing the practical examples 13 to 16, i.e. the mastic resin solution to 0.5%. As well, the bacterium number translates into common logarithm similar to Tables 1, 2 and 3, so that the figure length of the bacterium number becomes clear.

In the comparative examples 9 and 10, the bacterium number is gradually one digit decreasing or approximately constant, that is the variation of the number is not much, however, in the practical example 13 only containing 0.5% of the mastic resin solution and the practical example 14 containing 0.5% of the mastic resin solution and 0.03% of the mastic essential oil, the bacterium number of *P. gulae* is also only little reducing similar to the comparative examples 9 and 10. As well, the practical example 15 containing 0.5% of the mastic resin solution and 0.01% of the nano-type lactobacillus and the practical example 16 containing 0.5% of the mastic resin solution, 0.03% of the mastic essential oil and 0.01% of the nano-type lactobacillus had the results similar to the practical examples 13 and 14.

From this thing, when, practical examples 13-16, i.e. the mastic resin solution was set to 0.5%, there was the number of the bacteria of *P. gulae* in a tendency to decrease. However, it understood that the practical examples 13-16 are slightly reducing of the bacterium number or slightly constant different from the practical examples 1 to 8.

From the above, in the test example 1, although only *P. gulae* as a target, there are more consideration, whether the antibacterial (sterilization) action is shown to other periodontal disease bacteria particular to dog or cat (such as *Porphyromonas salivosa, Porphyromonas macacae,* and so on) or not and more consideration about sophisticated conditions, we thought that the antibacterial action and effect to the mastic resin solution, the mastic essential oil and/or the nano-type lactobacillus are the components of the oral composition for animal of the present invention are substantially shown in test tube (in *vitro*) scale at least.

[Preparation example 6] Preparations of periodontal disease bacteria (*P. gulae* (cat), *P. circumdentaria*) solution and infection disease bacteria (*Pas. multocida*) solution of cat.

As periodontal disease bacteria using in test example 2 mentioned later, three bacteria that are *Porphyromonas gulae* (hereinafter, so-called "P. *gulae* (cat)"; bacterial strein C26Db5) and *Porphyromonas circumdentaria* (hereinafter, so-called "*P*. *circumdentaria";* bacterial strein YC34b) of periodontal disease pathogen of cat and *Pasteurella multocida* (hereinafter, so-called "Pas. *multocida";* bacterial strein 12K) of infection disease pathogen were selected.

Next, *P. gulae* (cat) was anaerobically cultured at 37 °C and 5 days by using CDC (Centers for Disease Control and prevention) sheep blood agar medium for anaerobic bacteria (Oxioid. Made in Japan Becton, Dickinson and Company). *P. circumdentaria* was anaerobically cultured at 37 °C and 5 days by using blood agar medium adding 10.0mL/L of hemin and 10.0mL/L of menadione (mhTS). *Pas. multocida* was aerobically cultured at 37 °C and 1 day by using tryptic soy agar medium (TSA, Difco).

Then, with regard to the bacteria respectively culturing *P*. *gulae* (cat), *P. circumdentaria,* and *Pas. multocida,* the respective bacteria solutions were prepared by collecting suitable amount of growth colony of the respective bacteria by platinum loop while suspending the respective colony in sterilized saline (McFarland standards No.1 concentration). Hereinafter, the respective bacteria solutions were, corresponding to kinds of bacteria, periodontal disease (*P. gulae* (cat)) bacterium solution, periodontal disease (*P. circumdentaria*) bacterium solution and infection disease (*Pas. multocida*) bacterium solution. As well, the number of the respective bacteria is about 1~3×10⁸ CFU/mL.

### [Test Example 2] anti-periodontal disease bacterium effect test Part 2

The anti-periodontal disease bacteria effect was performed by using the periodontal disease *(P. gulae* (cat)) bacterium solution, the periodontal disease (*P*. *circumdentaria*) bacterium solution and the infection disease (*Pas. multocida*) bacterium solution, which were prepared in above Preparation Example 6, and a test solution for the anti-periodontal disease bacterium effect test (test solution). As well, the test solution for the anti-periodontal disease bacterium effect test (test solution) using in Test Example 2 was prepared so as to 5.0% of the mastic resin solution and 0.03% of the mastic essential oil in accordance with above Preparation Example 4. As well, a test solution in a control test ("control". Hereinafter, "control test solution") was also only tri(caprylic acid/capric acid) glyceryl without mixing the mastic resin solution and the mastic essential oil similar to above Preparation Example 4.

First, the bacterium number was chronologically measured by adding 1mL of the respective bacteria solutions (the periodontal disease (*P. gulae* (cat)) bacterium solution, the periodontal disease (*P. circumdentaria*) bacterium solution and the infection disease (*Pas. multocida*) bacterium solution) to 9mL of test solution.

With regard to the measurement of the bacterium number, ten times steps dilutions to the respective bacteria solutions were performed by repeatedly collecting 1mL of the mix solution between the test solution and the respective bacteria solutions after stirring immediately after adding the respective bacteria solutions to the test solution with stirring (hereinafter, reaction time 0 minute), further stirring with adding 9mL of the sterilized saline, and furthermore stirring with adding 9mL of the sterilized saline to 1mL of the diluted solution. After diluting, 0.1mL of the respective bacteria solutions was smeared on two blood agar mediums respectively. As well, with regard to the mediums, the periodontal disease (*P*. *gulae* (cat)) bacterium solution and the periodontal disease (*P. circumdentaria*) bacterium solution were anaerobically cultured at 37 °C, 5 days, and the infection disease (*Pas. multocida*) bacterium solution was aerobically cultured at 37 °C, 1 day. With regard to the respective bacteria solutions, the bacterium number in the test solution (the respective bacteria solutions mixed) was measured from the colony numbers of the respective mediums after cultivating. As well, the measured limit value was 3.5 (log[CFU/mL]), and the bacterium number below the measured limit value was described <3.5 (log[CFU/mL]).

Then, with regard to the test solutions of 1, 3, 5, 10 minute(s) or 5, 10, 15, 30 minutes after adding the respective bacteria solutions to the test solution with stirring, the bacterium number was measured after diluting at ten times steps by adding the sterilized saline to the respective test solutions similar to the method in case of reaction time 0 minute and cultivating. As control, the bacterium number was similarly and chronologically measured by adding the control test solution to the bacterium solution.

The results of the anti-periodontal disease bacterium effect test by mixing and stirring between the respective bacteria solution (the periodontal disease (*P*. *gulae* (cat)) bacterium solution, the periodontal disease (*P. circumdentaria*) bacterium solution and the infection disease (*Pas. multocida*) bacterium solution) and the test solution are respectively shown in Tables 5 to 7. "test section" in Tables 5 to 7 is the solution adding the respective bacteria solution to the test solution.

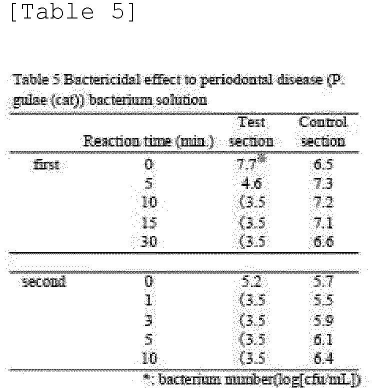

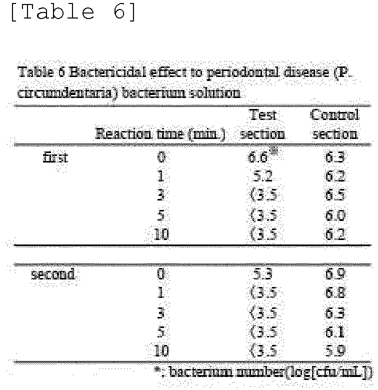

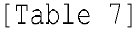

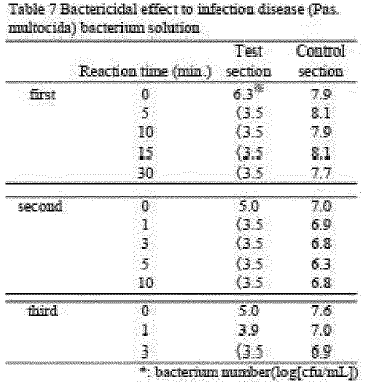

Table 5 shows a result of the anti-periodontal disease bacterium effect test between the periodontal disease *(P. gulae* (cat)) bacterium solution and the test solution. When adding to 7.7 (log[CFU/mL]) of the bacterium solution to the test solution, the bacterium number was 4.6 (log[CFU/mL]) after adding (strirring) 5 minutes and was below the measuring limit at 10 minutes.

On the other hand, in the control section, the bacterium number was substantially constant at about 7.0±0.5 (log[CFU/mL]) in 0~30 minutes after stirring. As well, when adding to 5.2 (log[CFU/mL]) of the bacterium solution to the test solution, the bacterium number was below the measuring limit after adding 1 minute.

Table 6 shows a result of the anti-periodontal disease bacterium effect test between the infection disease (*Pas.* circumdentaria bacterium solution) and the test solution. When adding to the bacterium solution to the test solution, the bacterium number was below the measuring limit after adding 1 minute or 3 minutes. On the other hand, in the control section, the bacterium number was 5.9~7.0 (log[CFU/mL]) in 0~30 minutes after stirring.

Table 7 shows a result of the anti-periodontal disease bacterium effect test between the infection disease (*Pas. multocida*) bacterium solution) and the test solution. When adding to the bacterium solution to the test solution, the bacterium number was 1% or less (about 4.0
(log[CFU/mL])) and was below the measuring limit after adding 3 minutes later. On the other hand, in the control section, the bacterium number was 6.3~8.1 (log[CFU/mL]) in 0~30 minutes after stirring.

In the test example, although the reaction conditions such as the bacterium number of the respective periodontal disease bacteria or the infection disease bacterium or the aerobic or anaerobic condition have dispersions, the oral composition for animal of the present invention could be found that the composition shows the bacteriocidal effects to *Porphyromonas gulae* derived from both dog and cat from the start, *Porphyromonas circumdentaria* which is other *Porphyromonas,* or *Pasteurella multocida* which is other genus.

### [Preparation Example] Preparation of an oral composition for animal of the present invention

The oral composition for animal of the present invention (gel paste) was prepared on the basis of the knowledge of the above test example. By the way, the preparation method of the composition used routine procedure. As well, with regard to the component ratio (weight %) of the respective components, it is just Table 8 shown in follows. As well, the gross weight of the oral composition was 25kg.

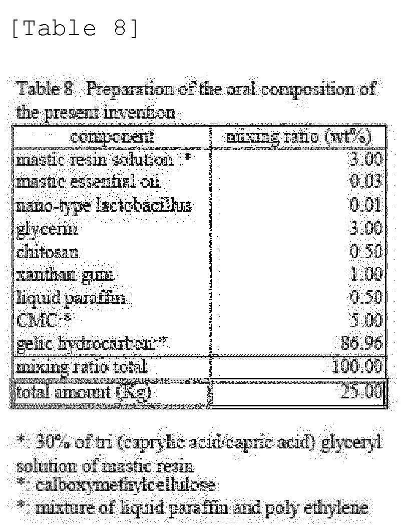

As well, with regard to the gel paste oral composition for animal prepared by the preparation example, its application is performed by coating the teeth or gums of animals (dog, cat) with soaking in toothbrush or swab.

As well, the above preparation example (charge-in quantity) is one example, and the other components can be suitably changed if following the composition such as the mastic resin (resin solution) and the mastic essential oil and/or the nano-type lactobacillus.

### [Test Example 3] Clinical test of the oral composition for animal

With regard to the gel paste, i.e., the oral composition for animal (hereinafter, "mastic gel") in the above preparation example, the clinical test of the oral composition for animal was performed to a household breeding dog (the dog species does not matter. Hereinafter, so-called simply "dog". ).

First, with regard to the household breeding dog, ten dogs, which completely have more than 0.5 of a determination score of a gingival index, which received a scaling (dental scaling) treatment, and which have less than 25 kg, are more than 3 years old and are without regard to sex, were suitable. Then, ten dogs were separated 5 dogs of control group (no coating of the mastic gel. However, usual dental cares were given except coating of the mastic gel.) and 5 dogs of test medicine group (the mastic gel were coated.). As well, the dogs both control group and test medicine group objected canines of the right and left and top and bottom jaws and fourth premolar tooth as teeth of testing object. Here, with regard to the calculation method for the determination score of the gingival index, the method will be shown in Table 9.

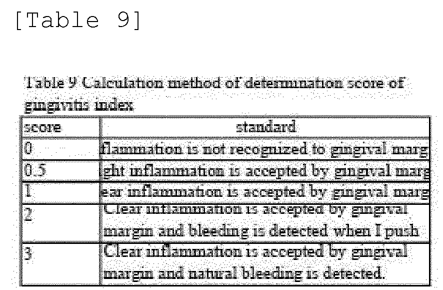

Next, a method for administering (coating and rubbing) the mastic gel to the dogs of the test medicine group was that an owner of the dog coated and rubbed about 0.5 g of the mastic gel to the respective gums of a left and top jaw, a left and bottom jaw, a right and top jaw, and a right and bottom jaw of the dog by the owner's finger. Then, the coating and rubbing of the mastic gel were performed once a day before sleeping and every day for weeks. As well, after the gel dosage to avoid the simultaneous combination of the supplement of the dental care purpose in the dosage of the mastic gel, we warned that the dogs put down a meal, water to drink for 30 minutes.

Next, an effect test of medical practice was performed. The effect test was performed as showing follow four tests: 1) the calculation of the determination score of the gingivitis index; 2) analysis of the periodontal bacteria and pilus type; 3) the calculation of the number of oral bacteria; and 4) a mouth odor measuring. Now, the respective tests will be explained in turn.

### [Test Example 3-1] Calculation of determination score of gingivitis index

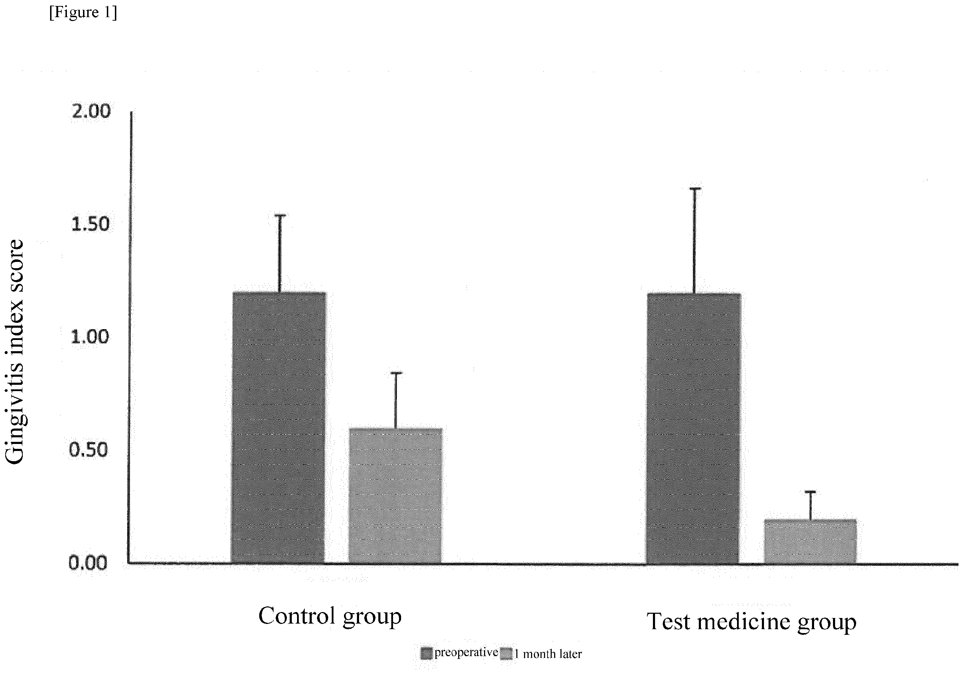

First, with regard to the calculation of determination score of gingivitis index, the determination score of gingivitis index to all dogs of the control group and the test medicine group in the scaling treatment before (hereinafter "preoperative") and the scaling treatment after 1 month (hereinafter "after 1 month") was judged by the observation by visual recognition on the basis of showing in Table 9. Further, here, there was effective in case that the determination score of the gingivitis index did not get worse than the preoperative or was improved. A transition of the determination score of the gingivitis index of the present Test Example 3-1 will be shown in Figure 1.

In Figure 1, in the control group and the test medicine group, there was difference in a value of the determination score of the gingivitis index between the scaling treatment before ("preoperative") and the scaling treatment after 1 month ("after 1 month") respectively. Furthermore, when the respective scaling treatments after 1 month ("after 1 month") was compered in the control group and the test medicine group, the test medicine group (the dogs which the mastic gel was used) had decreasing of the determination score, that is, an improvement of gingivitis was seen in the test medicine group. From this thing, although there is room for consideration with regard to statistical or detail, this indicated that the oral composition of the present invention shows the effect as gingivitis and thus periodontal disease PREVENTIVE.

### [Test Example 3-2] Analysis of the periodontal bacteria and pilus type

Next, analysis of the periodontal bacteria and pilus type will be explained.

First, the detection of the periodontal original bacteria was performed by using PCR (polymerase chain reaction) analysis method. The neighborhood of gingival margin on the fourth premolar tooth method side of right and left, the upper jaw of each dog concerned with both control group and test medicine group in the collection of periodontal disease original bacteria a swab (seed swab (trademark) γ 2. made by EIKEN CHEMICAL Co., Ltd. ). By the way, with regard to three pieces of swab, the PCR analysis was performed by extracting DNA of the bacteria (periodontal disease bacteria) from the collected swab sample, and the bacteria was detected by agarose gel electrophoretic analysis. By the way, fimA-type of the examined bacteria and *Porphyromonas gulae* is as follows: *Tannerella forsynthensis, Campylobacter rectus, Porphyromonas gingvalis, Porphyromonas gulae, Porphyromonas gulae* fimA A~C type. Then, fimA test (see WO2013/089166) was performed parallel to the PCR analysis. Further, the PCR analysis used Student t-test, Mann-Whitney U-test, Wilcoxon signed-rank test and Fisher's exact test, and there was statistical significance in case P-value<0.05.

The detection result of the periodontal disease bacteria by the PCR analysis and *Porphyromonas gulae* fimA test will be shown in Table 10.

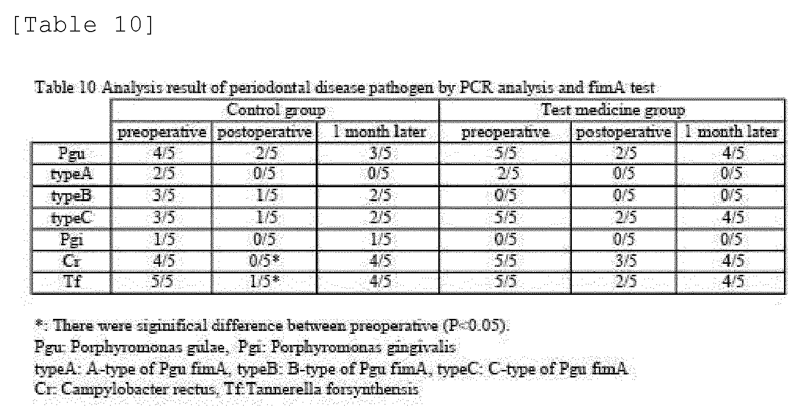

With regard to the PCR analysis, in the analyzed periodontal disease bacteria, both the control group and the test medicine group could be seen that the population detected after scaling reduced, and that the population 1 month later increased again (see Table 10).

With regard to the fimA test, although both groups holded A-type of *Porphyromonas gulae* fimA before scaling, the fimA disappeared in all dogs of both groups and also did not detect after 1 month. Although here was no clear difference between the control group and the test medicine group in C-type of *Porphyromonas gulae* fimA, the test medicine effect was unknown in B-type of *Porphyromonas gulae* fimA because there were no dogs holding the B-type fimA in the test medicine group before scaling.

### [Test Example 3-3] Calculation of the number of oral bacteria

With regard to the number of oral bacteria, a cheek side of fourth cheek tooth of the left top jaw of the dogs both the control group and the test medicine group was rubbed at three times by a white sterilized swab, whereby the bacteria on the swab was calcurated by using an automatically bacterial count device (DU-AA01NP-H. made by Panasonic health care CO., LTD.). Further, the calculation used Student t-test, Mann-Whitney U-test, Wilcoxon signed-rank test and Fisher's exact test similar to the PCR calculation in above Test Example 3-2, and there was statistical significance in case P-value<0.05.

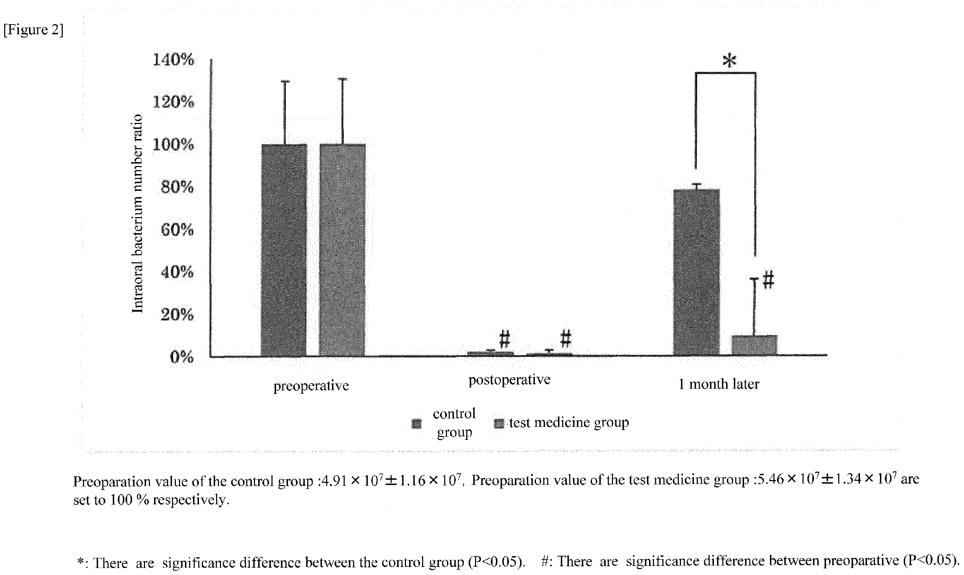

The figure showing the transition of the oral bacteria number will be shown in Figure 2. Further, "preoperative" in Figure 2 indicates before scaling treatment, "postoperative" indicates immediately after scaling treatment, and "1 month later" indicates after scaling treatment 1 month later. In Figure 2, the bacteria number significantly reduced by scaling in both the control group and the test medicine group (P<0.05). The bacteria number 1 month later in the test medicine group reduced more significantly than that one in the control medicine group and also significantly showed lower value than that one in preoperative.

### [Test Example 3-4] Mouth odor measuring

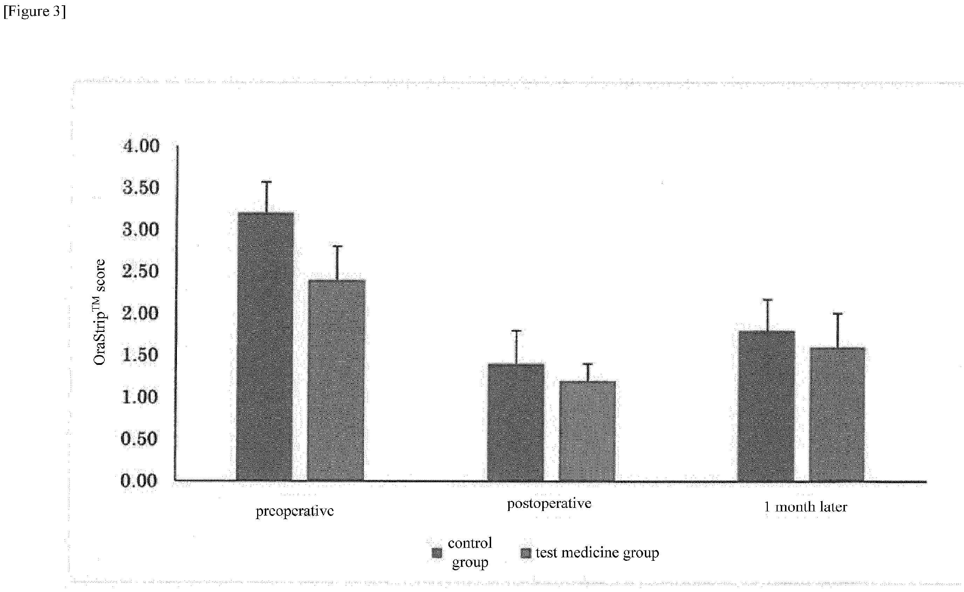

Next, a mouth odor measuring was performed by using OraStrip (Trademark. Made by DS Pharma Animal Health Co., Ltd.) toward the dogs of the control group and the test medicine group. The result will be shown in Figure 3. Further, "preoperative" in Figure 3 also indicates before scaling treatment, "postoperative" indicates immediately after scaling treatment, and "1 month later" indicates after scaling treatment 1 month later similar to Figure 2. In the test medicine group and the control group, an OraStrip score showed low value on both "preoperative" and "postoperative". Furthermore, in 1 month later, both groups showed lower value than "preoperative". As well, in the test medicine group and the control group, the OralStrip score of the test medicine group was lower value than the OralStrip score of the control group.

As the mentioned above, in Test Example 3 (3-1 to 3-4), although there would be the room of the various investigation, it was found that the mastic components have the applications as the periodontal (gingivitis) disease PREVENTIVE or the odor prevent agent of dog or cat by the clinical test using the oral composition of the present invention.

Although we explain the examples of the present oral composition for animal, the present inventions are not limited so the present examples are merely said examples.

### INDUSTRIAL APPLICABILITY OF THE PRESENT INVENTION

Although we explain about the oral composition for animal of the present invention, e.g. dental powder, in the above embodiments and examples, it is possible to apply as the antibacterial agent or the infection disease PREVENTIVE (e.g. toward parasitic bacteria of digestive organ) for animal in the oral composition for animal of the present invention because of using the mastic resin and/or essential oil.

## Claims

1. An oral composition for use in the prevention of a periodontal disease, an infection disease or mouth odor in an animal caused by a periodontal disease bacterium and an infection disease bacterium, **characterized in that** said oral composition contains mastic resin as an active component, said periodontal disease bacterium is *Porphyromonas circumdentaria,* and said infection disease bacterium is *Pasteurella multocida.*

2. The oral composition for use according to claim 1, wherein said mastic resin is used as a mastic resin solution having a concentration of 10-60 weight %.

3. The oral composition for use according to claim 2, wherein said mastic resin solution is made by solving in solvent, and wherein said solvent for solving said mastic resin is selected from any one of ethanol, glycerin, dipropylene glycol, 1,3-butylene glycol, fatty acid triglyceride (carbon numbers are 8-18 with regard to part of fatty acid derivation), tri(carpylic acid acid/capric acid) glyceryl, fatty acid monoglyceride (carbon numbers are 8-18 with regard to part of fatty acid derivation), mono carpylic acid glyceryl, fatty acid ester (carbon numbers are 8-18 with regard to part of fatty acid derivation), isopropyl myristate, ethyl iso-octanate, octyl dodecyl myristate, higher alcohol (carbon numbers are 8-18), oleyl alcohol, sorbitan fatty acid ester (carbon numbers are 8-18 with regard to part of fatty acid derivation) or sucrose fatty acid ester (carbon numbers are 8-18 with regard to part of fatty acid derivation).

4. The oral composition for use according to claims 2 or 3, wherein the content of said mastic resin solution is 0.1-50 weight % of the total amount of the oral composition.

5. The oral composition for use according to any one of claims 1 to 4, further containing mastic essential oil as an active component.

6. The oral composition for use according to claim 5, wherein the content of said mastic essential oil is 0.01-1.0 weight % of the total amount of the oral composition.

7. The oral composition for use according to any one of claims 1 to 6, wherein said animal is a dog or a cat.

8. The oral composition for use according to any one of claims 1 to 7, wherein a retaining agent is selected from one of the group of liquid paraffin, gelatinized hydrocarbon that is mixture of liquid paraffin and polyethylene, vegetable oil and yellow bees wax.

9. The oral composition for use according to any one of claims 1 to 8, wherein retaining agents are selected from two or more of the group of liquid paraffin, gelatinized hydrocarbon that is mixture of liquid paraffin and polyethylene, vegetable oil and yellow bees wax.

## Patentansprüche

1. Orale Zusammensetzung zur Verwendung bei der Vorbeugung einer Parodontitis, einer Infektionskrankheit oder Mundgeruch bei einem Tier, verursacht durch ein Parodontitis-Bakterium oder ein Bakterium einer Infektionskrankheit, **dadurch gekennzeichnet, dass** die orale Zusammensetzung Mastixharz als eine aktive Komponente umfasst, dass das Parodontitis-Bakterium *Porphyromonas circumdentaria* ist, und dass das Bakterium einer Infektionskrankheit *Pasteurella multocida* ist.

2. Orale Zusammensetzung zur Verwendung nach Anspruch 1, wobei das Mastixharz als eine Mastixharz-Lösung mit einer Konzentration von 10 bis 60 Gew.% verwendet wird.

3. Orale Zusammensetzung zur Verwendung nach Anspruch 2, wobei die Mastixharz-Lösung durch Auflösen in einem Lösungsmittel hergestellt ist, und wobei das Lösungsmittel zum Auflösen des Mastixharzes ausgewählt ist aus Ethanol, Glycerin, Dipropylenglycol, 1,3-Butylenglycol, Fettsäure-Triglyceride (8 bis 18 Kohlenstoffatome bezogen auf den Anteil der Fettsäurereste), Tri(caprylsäure/caprinsäure)glycerinester, Fettsäure-Monoglyceride (8 bis 18 Kohlenstoffatome bezogen auf den Anteil der Fettsäurereste), Monocaprylsäure-Glycerinester, Fettsäure-Ester (8 bis 18 Kohlenstoffatome bezogen auf den Anteil der Fettsäurereste), Isopropylmyristat, Ethyl-Iso-octanoat, Octyldodecyl-Myristat, höhere Alkohole (8 bis 18 Kohlenstoffatome), Oleylalkohol, Sorbitanfettsäureester (8 bis 18 Kohlenstoffatome bezogen auf den Anteil der Fettsäurereste) oder Saccharose-Fettsäureester (8 bis 18 Kohlenstoffatome bezogen auf den Anteil der Fettsäurereste).

4. Orale Zusammensetzung zur Verwendung nach Anspruch 2 oder 3, wobei der Anteil der Mastixharz-Lösung 0,1 bis 50 Gew.% der Gesamtmenge der oralen Zusammensetzung beträgt.

5. Orale Zusammensetzung zur Verwendung nach einem der Ansprüche 1 bis 4, ferner umfassend ätherisches Mastixöl als aktive Komponente.

6. Orale Zusammensetzung zur Verwendung nach Anspruch 5, wobei der Anteil des ätherischen Mastixöls 0,01 bis 1,0 Gew.% der Gesamtmenge der oralen Zusammensetzung beträgt.

7. Orale Zusammensetzung zur Verwendung nach einem der Ansprüche 1 bis 6, wobei das Tier ein Hund oder eine Katze ist.

8. Orale Zusammensetzung zur Verwendung nach einem der Ansprüche 1 bis 7, wobei ein Retentionsmittel ausgewählt ist aus der Gruppe umfassend flüssiges Paraffin, gelierte Kohlenwasserstoffe, d.h. Mischungen von flüssigem Paraffin und Polyethylen, pflanzliches Öl und gelbes Bienenwachs.

9. Orale Zusammensetzung zur Verwendung nach einem der Ansprüche 1 bis 8, wobei Retentionsmittel ausgewählt sind aus zwei oder mehreren aus der Gruppe umfassend flüssiges Paraffin, gelierte Kohlenwasserstoffe, d.h. Mischungen von flüssigem Paraffin und Polyethylen, pflanzliches Öl und gelbes Bienenwachs.

## Revendications

1. Composition orale pour utilisation dans la prévention d'une maladie parodontale, d'une maladie infectieuse ou d'une halitose chez un animal provoquée par une bactérie de maladie parodontale et une bactérie de maladie infectieuse, **caractérisée en ce que** ladite composition orale contient une résine de mastic en tant que composant actif, ladite bactérie de maladie parodontale est *Porphyromonas circumdentaria,* et ladite bactérie de maladie infectieuse est *Pasteurella multocida.*

2. Composition orale pour utilisation selon la revendication 1, dans laquelle ladite résine de mastic est utilisée sous forme de solution de résine de mastic présentant une concentration de 10-60 % en poids.

3. Composition orale pour utilisation selon la revendication 2, dans laquelle ladite solution de résine de mastic est obtenue par dissolution dans un solvant, et dans laquelle ledit solvant pour dissoudre ladite résine de mastic est l'un quelconque sélectionné parmi éthanol, glycérine, dipropylène glycol, 1,3-butylène glycol, triglycéride d'acide gras (les nombres de carbones sont de 8-18 pour une partie de dérivé d'acide gras), glycéryle de tri(acide caprylique/acide caprique), monoglycéride d'acide gras (les nombres de carbones sont de 8-18 pour une partie de dérivé d'acide gras), glycéryle de monoacide caprylique, ester d'acide gras (les nombres de carbones sont de 8-18 pour une partie de dérivé d'acide gras), myristate isopropylique, iso-octanate éthylique, myristate d'octyl-dodécyle, alcool supérieur (les nombres de carbones sont de 8-18), alcool oléique, ester d'acide gras et de sorbitan (les nombres de carbones sont de 8-18 pour une partie de dérivé d'acide gras) ou ester d'acide gras et de sucrose (les nombres de carbones sont de 8-18 pour une partie de dérivé d'acide gras).

4. Composition orale pour utilisation selon les revendications 2 ou 3, dans laquelle la teneur de ladite solution de résine de mastic est 0,1-50 % en poids de la quantité totale de la composition orale.

5. Composition orale pour utilisation selon l'une quelconque des revendications 1 à 4, contenant en outre une huile essentielle de mastic en tant que composant actif.

6. Composition orale pour utilisation selon la revendication 5, dans laquelle la teneur de ladite huile essentielle de mastic est 0,01-1,0 % en poids de la quantité totale de la composition orale.

7. Composition orale pour utilisation selon l'une quelconque des revendications 1 à 6, dans lequel ledit animal est un chien ou un chat.

8. Composition orale pour utilisation selon l'une quelconque des revendications 1 à 7, dans laquelle un agent de rétention est sélectionné parmi l'un du groupe d'une huile de paraffine, d'un hydrocarbure gélatinisé qui est un mélange d'huile de paraffine et de polyéthylène, d'une huile végétale et de la cire jaune d'abeille.

9. Composition orale pour utilisation selon l'une quelconque des revendications 1 à 8, dans laquelle des agents de rétention sont sélectionnés parmi au moins deux du groupe d'une huile de paraffine, d'un hydrocarbure gélatinisé qui est un mélange d'huile de paraffine et de polyéthylène, d'une huile végétale et de la cire jaune d'abeille.
